# EUROPEAN PATENT APPLICATION

(11) **EP 4 717 235 A2**
(43) Date of publication of application: **01.04.2026**
(21) Application number: 26150656.2
(22) Date of filing: 13.08.2024
(51) Int. Cl.: A61F 5/445

(54) **OSTOMY FILTER PROTECTION**

(30) Priority: 05.09.2023 US 202363580627 P
(62) Divisional of application: 24765813.1
(71) Applicant: Hollister Incorporated, Libertyville, IL 60048 (US)
(72) Inventor: WANG, Michelle, Libertyville, 60048 (US); BANNON, Peter, Libertyville, 60048 (US)
(74) Representative: FRKelly

(57) **Abstract**

An ostomy pouch appliance (10) includes a gas outlet (21) formed in one of the pouch walls for egress of gas collected in a pouch collection cavity, a filter assembly (22) attached to the pouch wall covering the gas outlet and a prefilter (24) configured to protect the filter assembly. The prefilter may have a crescent shaped body including pointed ends (28) configured to facilitate draining of body waste captured in the prefilter. The ostomy pouch appliance may also include a protective panel (20) configured to protect the filter assembly including at least one anti-reflux valve (130).

## Description

### BACKGROUND

The following description relates to an ostomy appliance, and more particularly, an ostomy pouch including a deodorizing filter and a filter protection.

Ostomy pouches for collecting body waste are used by patients who have had surgery such as a colostomy, ileostomy, or urostomy. An ostomy pouch includes an inlet opening for receiving body waste discharged from a stoma for collection within the pouch. Typically, the ostomy pouch also includes a gas outlet covered by a filter assembly for odor filtering and egress of gas collected in the pouch. However, in some instances, liquid, semisolid or solid body waste collected in the pouch may flow to and block the filter assembly, thereby restricting egress of gas through the filter assembly. This may lead to ballooning of the pouch or undesirable inflation of the pouch.

Disruptions in users' quality of life from the pouch ballooning can be significant including anxiety, lack of discretion, fear of leakage, inconvenient user intervention to release gas pressure, etc. Some common methods to release built-up gas include opening a pouch coupling system, which is often referred to as "burping", draining a pouch, and peeling back a skin barrier. Many ostomates have reported spending many hours troubleshooting pouch ballooning issues and feeling resigned about the current ostomy pouch systems.

Ostomy filter protection features have been developed to reduce the filter cogging problems. For example, US 2023/0099447, which is commonly assigned with the present application and incorporated herein in its entirety by reference, discloses an ostomy pouch including a filter assembly and a multi-stage filter protection, which includes a prefilter formed from a foam and a microperforated protective panel covering the prefilter.

The present disclosure provides improvements to the filter protection.

### BRIEF SUMMARY

An ostomy filter protection system is provided according to various embodiments.

In one aspect, the ostomy pouch appliance may include a body-side wall and a distal wall joined along an outer periphery defining a collection cavity for collecting body waste, inlet opening for receiving body waste from a stoma and a gas outlet formed in an upper portion of one of the body-side wall and distal wall for egress of gas collected in the collection cavity. The ostomy pouch appliance may also include a filter assembly attached to one of the body-side wall and distal wall covering the gas outlet and a prefilter configured to protect the filter assembly. The prefilter may have a crescent shaped body including pointed ends. The prefilter may be attached to one of the body-side wall and distal wall, such that the pointed ends are oriented downwardly toward a lower portion of the collection cavity to facilitate draining of body waste captured in the prefilter. The prefilter may be formed from a foam material.

In an embodiment, the filter assembly may be attached to an outer surface of one of the body-side wall and distal wall, and the prefilter may be attached to an inner surface of one of the body-side wall and distal wall covering the gas outlet.

In another embodiment, the filter assembly may be attached to an inner surface of one of the body-side wall and distal wall, and the prefilter may be attached to an inner surface of one of the body-side wall and distal wall covering the filter assembly.

In some embodiments, the ostomy pouch appliance may also include a protective panel formed from perforated film configured to protect the filter assembly and the prefilter. The protective panel may be attached to an inner surface of one of the body-side wall and distal wall covering the prefilter.

In an embodiment, the protective panel may include at least one anti-reflux valve. For example, the protective panel may include six anti-reflux valves. The at least one anti-reflux valve may be configured to open as the ostomy pouch appliance inflates to allow body waste captured between the protective panel and the pouch wall to drain down into the collection cavity and close as the ostomy pouch appliance deflates to prevent body waste collected in the collection cavity from flowing up toward the filter assembly. The at least one anti-reflux valve may be defined by curved slits formed in a lower portion of the protective film.

In an embodiment, the at least one anti-reflux valve may be backed by a foam material configured to function as a spring-loaded backing to keep the anti-reflux valve closed when the ostomy pouch appliance is deflated. For example, the at least one anti-reflux valve may be backed by an open-cell foam.

In another aspect, the ostomy pouch appliance may include a body-side wall and a distal wall joined along an outer periphery defining a collection cavity for collecting body waste, inlet opening for receiving body waste from a stoma and a gas outlet formed in an upper portion of one of the body-side wall and distal wall for egress of gas collected in the collection cavity. The ostomy pouch appliance may also include a filter assembly attached to one of the body-side wall and distal wall covering the gas outlet and a protecive panel configured to protect the filter assembly. The protective panel may include at least one anti-reflux valve configured to open as the ostomy pouch appliance inflates and close as the ostomy pouch appliance deflates. The protective panel may be formed from a perforated film. The at least one anti-reflux valve may be defined by curved slits formed in a lower portion of the protective film.

In an embodiment, the at least one anti-reflux valve may be backed by a foam material, such as an open-cell foam, configured to function as a spring-loaded backing to keep the anti-reflux valve closed when the ostomy pouch appliance is deflated. In an embodiment, the protective panel may include six anti-reflux valves.

The ostomy pouch appliance may further include a prefilter configured to protect the filter assembly. In an embodiment, the filter assembly may be attached to an outer surface of the pouch wall covering the gas outlet, and the prefilter may be attached to an inner surface of the pouch wall covering the gas outlet, and the protective panel may be attached to an inner surface of the pouch wall covering the prefilter. In another embodiment, the filter assembly may be attached to an inner surface of the pouch wall covering the gas outlet, and the prefilter may be attached to an inner surface of the pouch wall covering the filter assembly, and the protective panel may be attached to an inner surface of the pouch wall covering the prefilter. The prefilter may have a crescent shaped body including pointed ends and attached to the pouch wall such that the pointed ends are oriented downwardly toward a lower portion of the collection cavity to facilitate draining of body waste captured in the prefilter. In some embodiments, the prefilter may be formed from a foam material.

In some embodiments, the at least one anti-reflux valve may be arranged adjacent the prefilter formed by a foam material, such that the at least one anti-reflux valve is backed by the prefilter. In such an embodiment, the prefilter may function as a backing to keep the at least one anti-reflux valve closed when the ostomy pouch is deflated.

The foregoing general description and the following detailed description are examples only and are not restrictive of the present disclosure.

### BRIEF DESCRIPTION OF THE DRAWINGS

The benefits and advantages of the present embodiments will become more readily apparent to those of ordinary skill in the relevant art after reviewing the following detailed description and accompanying drawings, wherein:
FIG. 1 is a perspective distal view of an ostomy pouch appliance comprising a filter assembly and a multi-stage filter protection including a prefilter and a protective panel according to an embodiment;
FIG. 2 is a cross sectional view of the ostomy pouch appliance of FIG. 1;
FIG. 3 is a perspective view of a prefilter having a crescent shaped body according to an embodiment;
FIG. 4 is a partial exploded view of an ostomy pouch appliance comprising a filter assembly and a multi-stage filter protection according to an embodiment;
FIG. 5 is a perspective distal view of the ostomy pouch appliance of FIG. 4
FIG. 6 is a perspective distal view of the ostomy pouch appliance according to an embodiment; and
FIG. 7 is a cross sectional view of the ostomy pouch appliance of FIG. 6.

### DETAILED DESCRIPTION

While the present disclosure is susceptible of embodiment in various forms, there is shown in the drawings and will hereinafter be described presently preferred embodiments with the understanding that the present disclosure is to be considered an exemplification and is not intended to limit the disclosure to the specific embodiments illustrated. The words "a" or "an" are to be taken to include both the singular and the plural. Conversely, any reference to plural items shall, where appropriate, include the singular. The words "first," "second," "third," and the like may be used in the present disclosure to describe various information, such information should not be limited to these words. These words are only used to distinguish one category of information from another. The directional words "top," "bottom," up," "down," front," "back," and the like are used for purposes of illustration and as such, are not limiting. Depending on the context, the word "if" as used herein may be interpreted as "when" or "upon" or "in response to determining."

The present disclosure provides an ostomy filter protection system. The ostomy filter protections system may include a multi-stage system that can protect a filter from clogging or fouling by body waste or stoma effluent collected in the ostomy pouch. The ostomy filter protection system can include a perforated film and a prefilter foam element that can also act as a spacer between the perforated film and the distal side wall of the ostomy pouch to prevent gas from becoming trapped if the distal side wall and inner perforated film cling together. The prefilter can further have a shape for directing drainage of effluent away from the foam element. For example, the prefilter can be a crescent shaped foam element with ends that are oriented toward the pouch outlet opening.

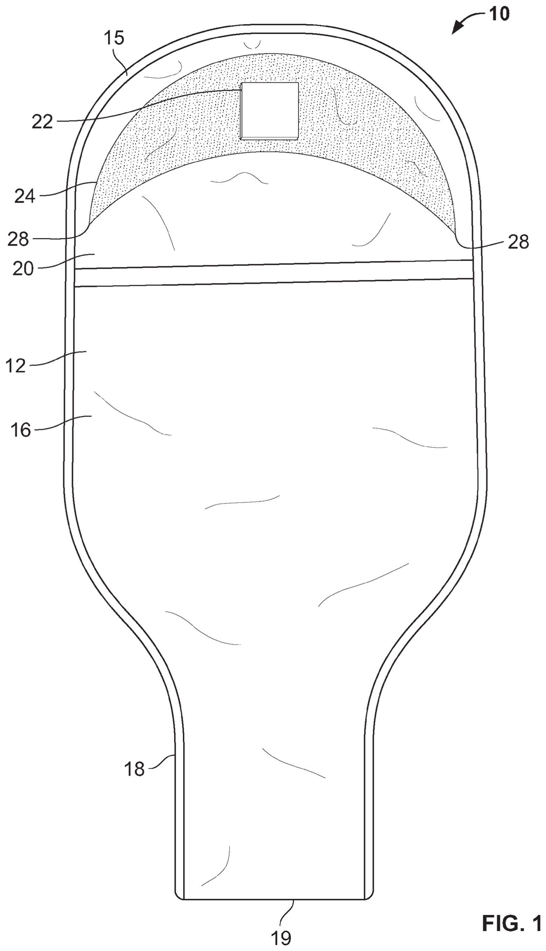

Referring now to the figures, FIGS. 1-2 illustrate an ostomy pouch appliance 10 according to an embodiment. The ostomy pouch appliance 10 may be formed by a distal wall 12 and a body side wall 14 joined together along an outer peripheral seal 15 to define a collection cavity 16 for collecting body waste and outlet portion 18. The ostomy pouch appliance 10 may include a filter assembly 22 covering a gas outlet opening 21 and a multi-stage filter protection comprising a protective panel 20 and a prefilter 24. The outlet portion 18 can terminate in a discharge opening or outlet 19 for draining the body waste collected in the cavity 16 after a period of use.

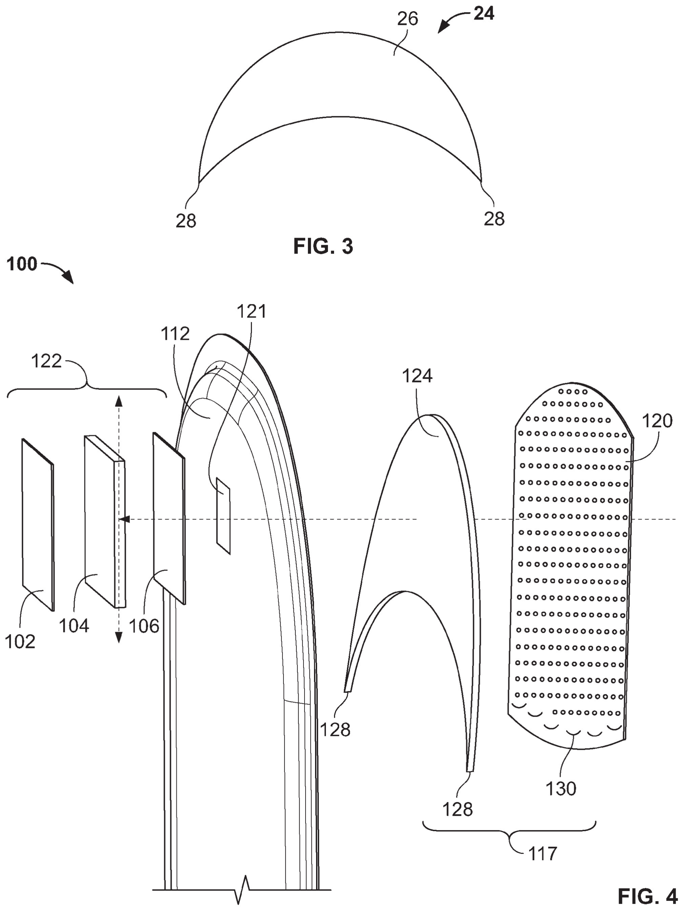

FIG. 3 shows the prefilter 24 according to an embodiment. The prefilter 24 may be configured to have a crescent shaped body including a central portion 26 and two ends 28 oriented towards the outlet portion 18 of the pouch 10 as best shown in FIG. 1. Suitable materials for the prefilter 24 may include, but are not limited to, foams, such as a reticulated open cell foam, textiles, microfleece, nonwoven, and the like. In an embodiment, the prefilter 24 may be formed from a foam material that, along with the protective panel 20, can provide a multi-stage filter protection to prevent or reduce the filter assembly 22 from clogging or fouling by body waste collected in the pouch.

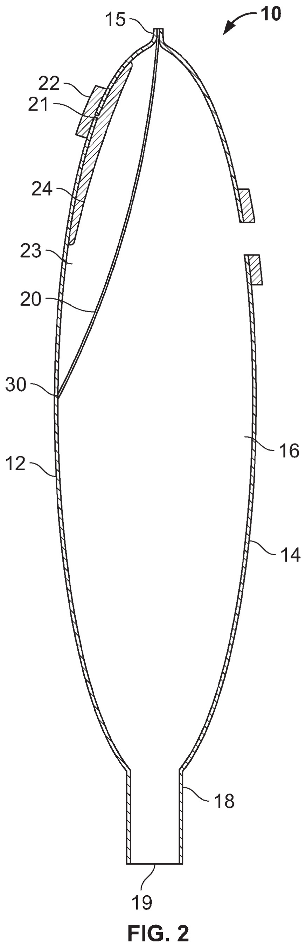

In the embodiment of FIG. 2, the filter assembly 22 is attached to an outer surface of the distal pouch wall 12 covering a gas outlet opening 21 defined therein, and the prefilter 24 is attached to an inner surface of the distal pouch wall 12 covering the gas outlet opening 21. In another embodiment, the filter assembly 22 may be attached to an inner surface of a pouch wall covering a gas outlet opening, and the prefilter 24 may be attached an inner surface of the pouch wall to cover the filter assembly 22. The prefilter 24 may be arranged such that the ends 28 of the prefilter 24 are oriented downwardly towards the outlet portion 18 to direct drainage of effluent away from the prefilter 24 as best shown in FIG. 1. The ends 28 of the prefilter 24 may be configured as pointed ends as best shown in FIG. 3 to facilitate drainage of body waste captured in the prefilter 24 towards the collection cavity 16. The prefilter 24 can also function as a spacer between the distal pouch wall 12 and the protective panel 20 to prevent the protective panel 20 and distal pouch wall 12 from clinging together and obstructing gas flow paths.

The protective panel 20 may be joined with the pouch walls 12, 14 along the peripheral seal 15 and attached to the distal wall 12 along a lower seal 30 to define a filter protection cavity 23 as shown in FIGS. 1 and 2. The protective panel 20 may be formed from a perforated film configured to block ostomy effluent (also referred to herein as body waste, liquid body waste, semi solid body waste, and solid body waste) while allowing gas to flow therethrough. In an embodiment, the protective panel 20 may comprise a plurality of microperforations having a micro unit sized perforations. In some embodiments, the protective panel 20 may comprise perforations of various sizes and/or perforations in various patterns. For example, the protective panel 20 may comprise microperforations in a lower portion and may be free of perforation in an upper portion proximate the filter assembly 22.

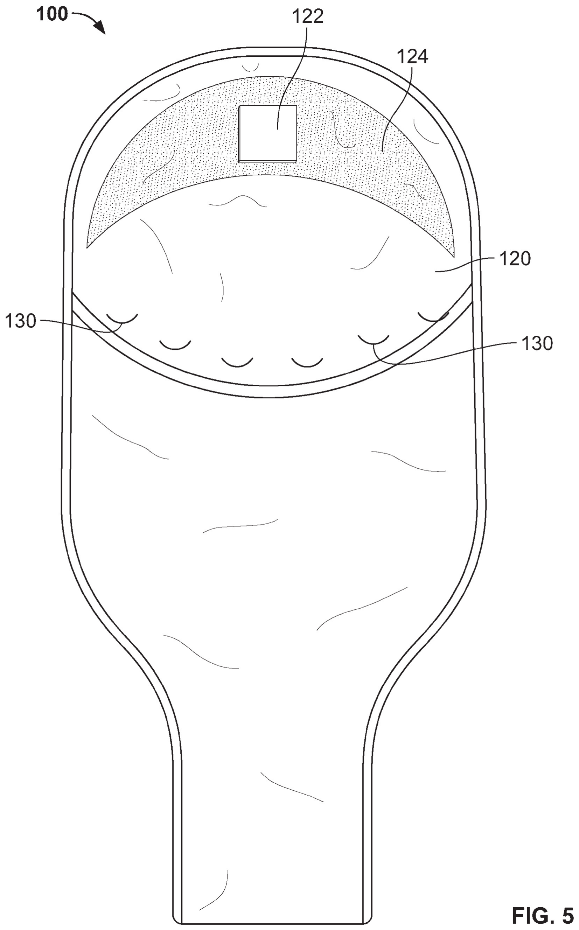

FIGS. 4 and 5 show an ostomy pouch appliance 100 according to another embodiment. FIG. 4 is a partial exploded view of the ostomy pouch appliance 100. The ostomy pouch appliance 100 may be configured similar to the ostomy pouch appliance 10 generally comprising a filter assembly 122 and a multi-stage protection 117 including a protective panel 120 and a crescent shaped prefilter 124. In this embodiment, the filter assembly 122 may be attached to an outer surface of a pouch wall 112 while the multi-stage protection 117 may be arranged inside the pouch. In another embodiment, the filter assembly 122 and the multi-stage protection 117 may both be arranged inside the pouch such that the filter assembly 122 covers a gas outlet opening 121 and the multistage protection 117 covers the filter assembly 122.

The filter assembly 122 may comprise a backing layer 102, a filter media 104, and a membrane layer 106. The filter assembly 122 may be arranged to cover the gas outlet opening 121 defined in the pouch wall 112 and attached to the pouch wall 112, for example via heat sealing. The membrane layer 106 may be formed from a suitable gas permeable material configured to allow gas to flow therethrough while providing protection against ostomy effluent. The filter media 104 may be formed from a suitable filter material configured to deodorize ostomy gas. The backing layer 102 may be formed from a suitable material that has a relatively low gas permeability or gas impermeable and configured to direct gas to flow radially through the filter assembly 416. In this embodiment, gas collected in the ostomy pouch appliance 100 may egress through the protective panel 120, the prefilter 124 and the outlet opening 121, and flow into the filter assembly 122 through the membrane layer 106 and radially flow through the filter media 104 before exiting the filter assembly 122 as indicated by arrows in FIG. 4. The filter assembly 122 may comprise at least one gas outlet proximate a periphery of the filter assembly 122. For example, the filter assembly 122 may comprise a gas outlet defined by an unsealed periphery.

The multi-stage filter protection 117 may comprise the prefilter 124 arranged to cover the gas outlet opening 121 and sealed to an inner surface of the pouch wall 112 and the protective panel 120 covering the prefilter 124 and sealed to an inner surface of the pouch wall 112. In such an embodiment, the protective panel 120 may function as a coarse prefilter and a first line of protection and the prefilter 124 may function as a fine prefilter and a second line of protection to provide a multiple protection for the filter assembly 122 from ostomy effluent collected in the pouch. In this embodiment, the prefilter 124 may be configured similar to the prefilter 24 having a crescent shaped body including two pointed ends 128 to facilitate draining of ostomy effluent captured in the prefilter 124 towards the pouch collection cavity.

The protective panel 120 may be formed from a suitable microperforated film and sealed to the pouch wall 112 via a peripheral seal. The protective panel 120 may be configured and sized larger than the prefilter 124 to cover and seal around the prefilter 124.

In an embodiment, the protective panel 120 may include anti-reflux valves 130. The anti-reflux valves 130 may be defined by curved slits formed in a lower portion of the protective panel as shown in FIG. 4. The anti-reflux valves 130 may be configured as dynamic valves that open as the pouch inflates and close as the pouch deflates. In an embodiment, the multi-stage filter protection 117 may be configured such that when the pouch becomes inflated, the protective panel 120 separates from the pouch wall 112 and the anti-reflux valves 130 open to allow ostomy effluent captured between the protective panel 120 and the pouch wall 112 to drain down into the pouch collection cavity. When the pouch is deflated, the anti-reflux valves 130 may close to prevent ostomy effluent collected in the pouch collection cavity from flowing up toward the filter assembly 122. In an embodiment, the protective panel 120 may include six anti-reflux valves 130. In some embodiments, the anti-reflux valves may be backed by a foam material, such as an open-cell foam, which may function as a spring-loaded backing to keep the valves closed when the pouch is deflated.

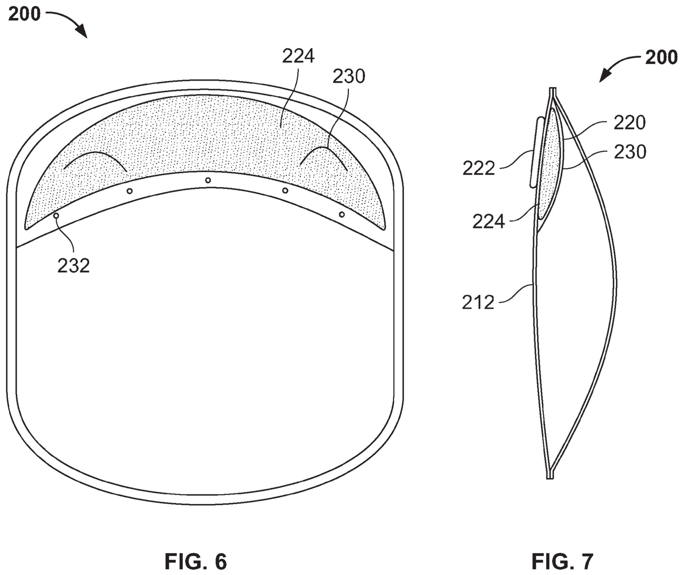

FIGS. 6 and 7 show an ostomy pouch appliance 200 according to yet another embodiment. The ostomy pouch appliance 200 may be configured similar to the ostomy pouch appliance 100 generally comprising a filter assembly 222 and a multi-stage protection including a protective panel 220 and a crescent shaped prefilter 224. The protective panel 220 may include anti-reflux valves 230 defined by curved slits formed in the protective panel 220. The anti-reflux valves 230 may be configured as dynamic valves that open as the pouch inflates and close as the pouch deflates. In this embodiments, the anti-reflux valves 230 may be arranged adjacent the crescent shaped prefilter 224, such that the anti-reflux valves 230 may be backed by the crescent shaped prefilter 224. In such an embodiment, the crescent shaped prefilter 224 may be formed from a foam material and function as a backing for the anti-reflux valves 230 to keep the anti-reflux valves 230 closed when the pouch is deflated. In the embodiment of FIG. 6, the protective panel 220 include two anti-reflux valves 230. In other embodiments, the protective panel 220 may include more than two anti-reflux valves. In an embodiment, the protective panel 220 may include a plurality of holes 232 to provide additional paths for ostomy effluent captured between the protective panel 220 and a pouch wall 212 to drain down into the pouch collection cavity.

From the foregoing it will be observed that numerous modifications and variations can be effectuated without departing from the true spirit and scope of the novel concepts of the present disclosure. It is to be understood that no limitation with respect to the specific embodiments illustrated is intended or should be inferred. The disclosure is intended to cover by the appended claims all such modifications as fall within the scope of the claims.

Furthermore, one or more of the following numbered clauses may describe and relate to further aspects or features within the context of the present teaching:
1. An ostomy pouch appliance comprising: a body-side wall and a distal wall joined along an outer periphery defining a collection cavity for collecting body waste; inlet opening for receiving body waste from a stoma; a gas outlet formed in an upper portion of one of the body-side wall and distal wall for egress of gas collected in the collection cavity; a filter assembly attached to one of the body-side wall and distal wall covering the gas outlet; and a prefilter configured to protect the filter assembly and attached to one of the body-side wall and distal wall, wherein the prefilter has a crescent shaped body including pointed ends configured to facilitate draining of body waste captured in the prefilter.
2. The ostomy pouch appliance of clause 1, wherein the prefilter is arranged such that the pointed ends are oriented downwardly toward a lower portion of the collection cavity.
3. The ostomy pouch appliance of any of clauses 1-2, wherein the filter assembly is attached to an outer surface of one of the body-side wall and distal wall, and the prefilter is attached to an inner surface of one of the body-side wall and distal wall covering the gas outlet.
4. The ostomy pouch appliance of any of clauses 1-2, wherein the filter assembly is attached to an inner surface of one of the body-side wall and distal wall, and the prefilter is attached to an inner surface of one of the body-side wall and distal wall covering the filter assembly.
5. The ostomy pouch appliance of any of clauses 1-4, wherein the prefilter is formed from a foam material.
6. The ostomy pouch appliance of any of clauses 1-5, further including a protective panel formed from perforated film, wherein the protective panel is configured to protect the filter assembly and the prefilter.
7. The ostomy pouch appliance of clause 6, wherein the protective panel is attached to an inner surface of one of the body-side wall and distal wall covering the prefilter.
8. The ostomy pouch appliance of any of clauses 6-7, wherein the protective panel includes at least one anti-reflux valve, wherein the at least one anti-reflux valve is configured to open as the ostomy pouch appliance inflates to allow body waste captured between the protective panel and one of the body-side wall and distal wall to drain down into the collection cavity, and wherein the at least one anti-reflux valve is configured to close as the ostomy pouch appliance deflates to prevent body waste collected in the collection cavity from flowing up toward the filter assembly.
9. The ostomy pouch appliance of clause 8, wherein the at least one anti-reflux valve is defined by curved slits formed in a lower portion of the protective film.
10. The ostomy pouch appliance of any of clauses 8-9, wherein the at least one antireflux valve is backed by a foam material configured to function as a spring-loaded backing to keep the anti-reflux valve closed when the ostomy pouch appliance is deflated.
11. The ostomy pouch appliance of any of clauses 8-9, wherein the at least one antireflux valve is arranged adjacent the prefilter formed by a foam material, such that the at least one anti-reflux valve is backed by the prefilter, wherein the prefilter functions as a backing to keep the at least one anti-reflux valve closed when the ostomy pouch appliance is deflated.
12. The ostomy pouch appliance of any of clams 6-11, wherein the protective panel includes six anti-reflux valves.
13. An ostomy pouch appliance comprising: a body-side wall and a distal wall joined along an outer periphery defining a collection cavity for collecting body waste; inlet opening for receiving body waste from a stoma; a gas outlet formed in an upper portion of one of the body-side wall and distal wall for egress of gas collected in the collection cavity; a filter assembly attached to one of the body-side wall and distal wall covering the gas outlet; and a protective panel configured to protect the filter assembly and attached to one of the bodyside wall and distal wall, wherein the protective panel includes at least one anti-reflux valve, wherein the at least one anti-reflux valve is configured to open as the ostomy pouch appliance inflates and close as the ostomy pouch appliance deflates.
14. The ostomy pouch appliance of clause 13, wherein the protective panel is formed from a perforated film.
15. The ostomy pouch appliance of any of clauses 13-14, wherein the at least one antireflux valve is defined by curved slits formed in a lower portion of the protective film.
16. The ostomy pouch appliance of any of clauses 13-15, wherein the at least one antireflux valve is backed by a foam material configured to function as a spring-loaded backing to keep the anti-reflux valve closed when the ostomy pouch appliance is deflated.
17. The ostomy pouch appliance of clause 16, wherein the at least one anti -reflux valve is backed by an open-cell foam.
18. The ostomy pouch appliance of any of clauses 13-17, wherein the protective panel includes six anti-reflux valves.
19. The ostomy pouch appliances of any of clauses 13-18, further including a prefilter configured to protect the filter assembly, wherein the filter assembly is attached to an outer surface of one of the body-side wall and distal wall covering the gas outlet, and the prefilter is attached to an inner surface of one of the body-side wall and distal wall covering the gas outlet, and the protective panel is attached to an inner surface of one of the body-side wall and distal wall covering the prefilter.
20. The ostomy pouch appliances of any of clauses 13-18, further including a prefilter configured to protect the filter assembly, wherein the filter assembly is attached to an inner surface of one of the body-side wall and distal wall covering the gas outlet, and the prefilter is attached to an inner surface of one of the body-side wall and distal wall covering the filter assembly, and the protective panel is attached to an inner surface of one of the body-side wall and distal wall covering the prefilter.
21. The ostomy pouch appliances of any of clauses 19-20, wherein the prefilter has a crescent shaped body including pointed ends configured to facilitate draining of body waste captured in the prefilter, wherein the prefilter is arranged such that the pointed ends are oriented downwardly toward a lower portion of the collection cavity.
22. The ostomy pouch appliance of any of clauses 19-21, wherein the prefilter is formed from a foam material.
23. The ostomy pouch appliance of clause 22, wherein the at least one anti -reflux valve is arranged adjacent the prefilter, such that the at least one anti-reflux valve is backed by the prefilter, wherein the prefilter functions as a backing to keep the at least one anti -reflux valve closed when the ostomy pouch appliance is deflated.

## Claims

1. An ostomy pouch appliance comprising: a body-side wall and a distal wall joined along an outer periphery defining a collection cavity for collecting body waste;
inlet opening for receiving body waste from a stoma;
a gas outlet formed in an upper portion of one of the body-side wall and distal wall for egress of gas collected in the collection cavity;
a filter assembly attached to one of the body-side wall and distal wall covering the gas outlet; and
a protective panel configured to protect the filter assembly and attached to one of the bodyside wall and distal wall, wherein the protective panel includes at least one anti-reflux valve, wherein the at least one anti-reflux valve is configured to open as the ostomy pouch appliance inflates and close as the ostomy pouch appliance deflates.

2. The ostomy pouch appliance of claim 13, wherein the protective panel is formed from a perforated film.

3. The ostomy pouch appliance of any of claims 1-2, wherein the at least one antireflux valve is defined by curved slits formed in a lower portion of the protective film.

4. The ostomy pouch appliance of any of claims 1-3, wherein the at least one antireflux valve is backed by a foam material configured to function as a spring-loaded backing to keep the anti-reflux valve closed when the ostomy pouch appliance is deflated.

5. The ostomy pouch appliance of claim 4, wherein the at least one anti -reflux valve is backed by an open-cell foam.

6. The ostomy pouch appliance of any of claims 1-5, wherein the protective panel includes six anti-reflux valves.

7. The ostomy pouch appliances of any of claims 1-6, further including a prefilter configured to protect the filter assembly, wherein the filter assembly is attached to an outer surface of one of the body-side wall and distal wall covering the gas outlet, and the prefilter is attached to an inner surface of one of the body-side wall and distal wall covering the gas outlet, and the protective panel is attached to an inner surface of one of the body-side wall and distal wall covering the prefilter.

8. The ostomy pouch appliances of any of claims 1-6, further including a prefilter configured to protect the filter assembly, wherein the filter assembly is attached to an inner surface of one of the body-side wall and distal wall covering the gas outlet, and the prefilter is attached to an inner surface of one of the body-side wall and distal wall covering the filter assembly, and the protective panel is attached to an inner surface of one of the body-side wall and distal wall covering the prefilter.

9. The ostomy pouch appliances of any of claims 7-8, wherein the prefilter has a crescent shaped body including pointed ends configured to facilitate draining of body waste captured in the prefilter, wherein the prefilter is arranged such that the pointed ends are oriented downwardly toward a lower portion of the collection cavity.

10. The ostomy pouch appliance of any of claims 7-9, wherein the prefilter is formed from a foam material.

11. The ostomy pouch appliance of claim 10, wherein the at least one anti -reflux valve is arranged adjacent the prefilter, such that the at least one anti-reflux valve is backed by the prefilter, wherein the prefilter functions as a backing to keep the at least one anti -reflux valve closed when the ostomy pouch appliance is deflated.
